# EUROPEAN PATENT SPECIFICATION

(11) **EP 2 517 742 B1**
(45) Date of publication and mention of the grant of the patent: **18.05.2016**
(21) Application number: 12168941.8
(22) Date of filing: 24.12.2007
(51) Int. Cl.: A61M 1/28

(54) **Peritoneal dialysis therapy validation**
Peritonealdialyse-Behandlungsbewertung
Validation de thérapie de dialyse péritonéale

(30) Priority: 29.12.2006 US 648154
(43) Date of publication of application: 31.10.2012
(62) Divisional of application: 07025089.9
(73) Proprietor: Fresenius Medical Care Holdings, Inc., Waltham, MA 02451-1457 (US)
(72) Inventor: Kulwinder, S. Plahey, Martinez, CA California 94553 (US); Ly, Tri, Dublin, CA California 94568 (US); Pandurangi, Rashmi, Concord, CA 94518 (US); Zhu, Jie, Antioch, CA94509 (US)
(74) Representative: Conroy, John

(56) References cited:
- US-A- 5 989 423

## Description

### TECHNICAL FIELD

This disclosure relates to peritoneal dialysis therapy validation. A prior art example is disclosed in US5,989,423A.

### BACKGROUND

Peritoneal dialysis is a treatment for kidney failure that involves filling a patient's peritoneal cavity with a dialysis solution that aids in removing waste products from the body and later draining that solution. The filling and draining is handled by a device known as a cycler, such as the Newton™ IQ and the forthcoming Liberty™ Cycler from Fresenius Medical Care N.A. and the HomeChoice™ from Baxter Healthcare. Four types of peritoneal dialysis are discussed in this disclosure: continuous cycling peritoneal dialysis, intermittent peritoneal dialysis, PD plus, and tidal peritoneal dialysis.

Continuous cycling peritoneal dialysis (CCPD) is a continuous therapy. CCPD is the most common cycling therapy prescribed. With CCPD, the patient has dialysis solution in peritoneum at all times but the exchanges are done only at night by the cycler. Several exchanges are done during sleep. The last thing that the cycler will do is fill for the day. When reconnecting to the cycler, the dialysate that has been dwelling in the patient during the day first has to be drained.

Intermittent peritoneal dialysis (IPD), as its name suggests, is an interrupted or intermittent therapy. With IPD, the patient will receive exchanges every night from the cycler while sleeping, but will not have any dialysis solution in peritoneum during the day.

PD plus therapy (PD+) is a continuous therapy in which one or more exchanges are received during the day from the cycler, in addition to the nighttime exchanges. These daytime exchanges are called pause exchanges. With PD plus therapy, the patient carries dialysis solution in peritoneum during the day. This allows for continuous waste product and fluid removal.

Tidal peritoneal dialysis (Tidal) differs from other dialysis therapies in the way that dialysis solution is delivered during the nighttime. With Tidal the patient is filled with a prescribed amount of solution, then only a portion is drained and refilled with seach exchange. Depending on the prescription, the treatment may end with a fill or a drain.

### SUMMARY

The invention is defined in the appended claims. In general, in one aspect, parameter values are set in a peritoneal dialysis device by receiving from a user a selection of a therapy type, a value for an input parameter is received from the user, and a value is set for a number of fills parameter or a dwell time parameter based on the value received from the user.

Implementation may include one or more of the following features. Setting a value for the number of fills or dwell time parameter includes calculating a value for the number of fills or dwell time, and assigning the calculated value to the parameter. Setting a value for the number of fills or dwell time parameter includes calculating an updated value for the number of fills or dwell time, determining that the updated value does not meet a criterion, and assigning a value to a third parameter. Calculating an updated value for the number of fills or dwell time based on the value assigned to the third parameter, and assigning the updated value to the number of fills or dwell time parameter. The criterion is that the updated value is within a pre-set range. The criterion is that the updated value has a specified relationship to a fourth parameter. Communicating the value set for the number of fills or dwell time parameter to the user, and communicating to the user a relationship between the value received from the user and the value set for the number of fills or dwell time parameter.

In general, in one aspect, parameter values are set in a peritoneal dialysis device by receiving from a user a selection of a therapy type, receiving from a user values for a plurality of parameters, calculating a value for a plurality of additional parameters based on the values received from the user, the plurality of additional parameters comprising a number of fills parameter or a dwell time parameter, determining that values for one or more of the plurality of parameters or one or more of the additional parameters do not meet one or more criteria, and updating one or more of the values received from the user so that all of the values for the plurality of parameters and the additional parameters meet all the criteria.

In general, in one aspect, parameter values are set in a peritoneal dialysis device by displaying on a graphical interface a plurality of parameters and any values currently assigned to the parameters, in response to a user selecting one of the displayed parameters, allowing the user to input a value to be assigned to the parameter, calculating updated values for one or more parameters based on the value input by the user and assigning the updated values to the corresponding parameters, determining whether values assigned to one or more of the parameters meet one or more criteria, and, if the values do not meet the criteria, calculating one or more also updated values for other parameter values that will cause a value that does not meet a criterion to meet the criterion and assigning the also updated values to the corresponding parameters.

In general, in one aspect, parameter values are set in a peritoneal dialysis device by receiving an instruction from a user to change a value of a parameter to a new value, determining whether the new value is greater than a maximum value for the parameter, if the new value is greater than the maximum value, rejecting the new value, if the new value is less than the maximum value, determining whether the new value is less than a minimum value for the parameter, and if the new value is less than the minimum value, rejecting the new value.

Implementations may include, calculating an updated value for a second parameter based on the new value if the new value is greater than the minimum value, calculating a total fill volume, determining whether the total fill volume is greater than a total treatment volume, if the total fill volume is greater than the total treatment volume, rejecting the new value, and if the total fill volume is less than the total treatment volume, changing the value of the parameter to the new value.

In general, in some aspects, a peritoneal dialysis device displays on a graphical interface a plurality of parameters and any values currently assigned to the parameters, and in response to a user selecting one of the displayed parameters, allows the user to input a value to be assigned to one of the parameters. The device calculates updated values for one or more additional parameters based on the value input by the user and assigns the updated values to the corresponding parameters, determines whether values assigned to one or more of the additional parameters meet one or more criteria, and if the values do not meet the criteria, calculates one or more further updated values for other parameter values that will cause a value that does not meet a criterion to meet the criterion and assign the further updated values to the corresponding parameters

Implementations may include one or more of the following features. The device displays the updated values on the graphical interface. The device determines that values assigned to one or more of the additional parameters do not meet one or more criteria, and informs the user that the input value is rejected. The device performs peritoneal dialysis fills and drains based on the values assigned to the parameters. The device determines that the value input by the user does not meet one or more criteria, and informs the user that the input value is rejected. The graphical interface is part of the peritoneal dialysis device. The graphical interface includes a touch-sensitive display screen.

Other features and advantages of the invention will be apparent from the description and the claims.

### BRIEF DESCRIPTION OF THE FIGURES

FIGs. 1A and B are perspective views of a cycler.
FIG. 2 is a plan view of a cycler cartridge.
FIG. 3 is a block diagram of a control computer for a cycler.
FIGs 4A-F show user interface screens.
FIGs. 5A-B and 6A-B are flow charts of a parameter update process.
FIGs. 7A-D are graphs of dialysis solution amounts.
FIGs. 8A-B are tables of validation rules.
FIG. 9 is a block diagram of validation relationships.
FIGs. 10A-10D are flow charts of a validation process.
FIGs. 11A-11B are tables of parameter values.

### DETAILED DESCRIPTION

In current dialysis machines, the therapies are not customized to the specific dialysis type, e.g., CCPD, IPD, PD+, or Tidal. The user creates a therapy scenario himself based on values he chooses for such parameters as the total volume of solution to use, how much to use per fill, or how many pauses to have. Setting large numbers of parameters and keeping track of their interdependencies can be overwhelming. Some of this difficulty can be overcome by providing a user interface that enables a user to select from one of the pre-defined therapy types and then customize it to his prescription, with the system updating dependent and interdependent values as the user changes the ones under his control.

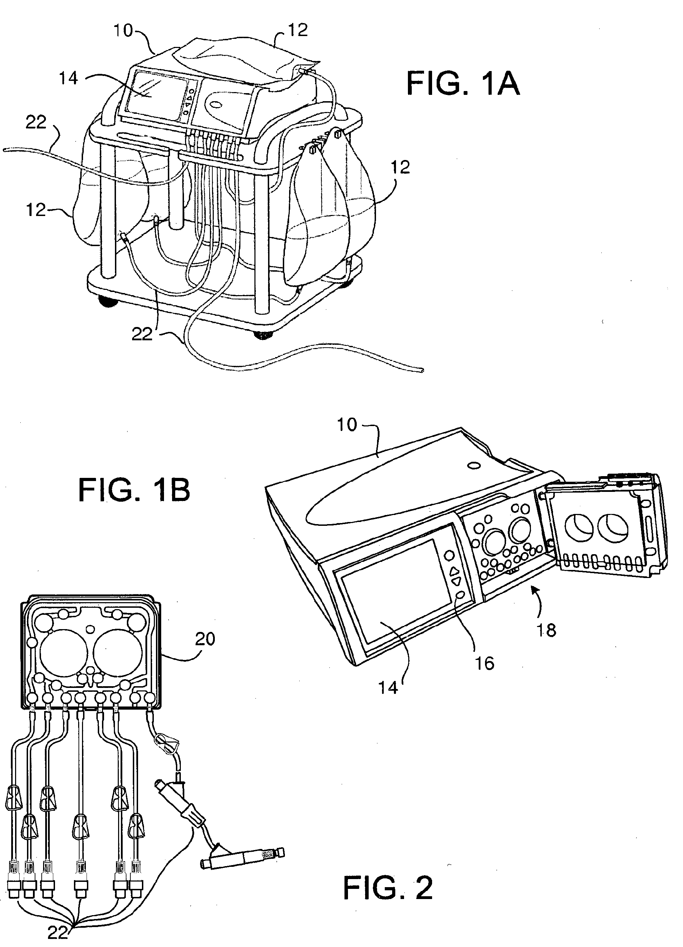

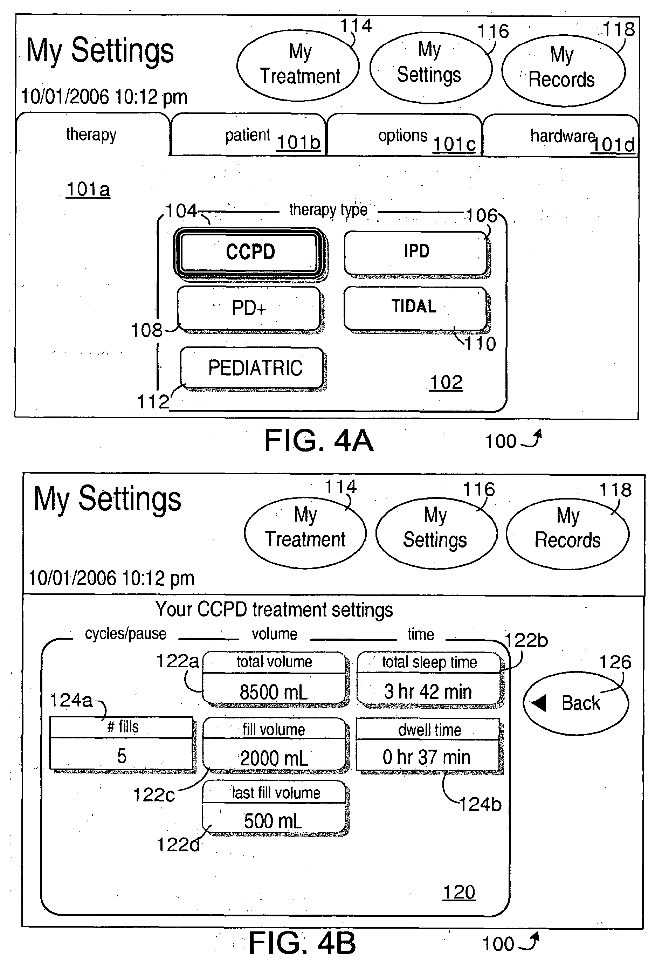

The following description relates to a prototype of the Liberty™ Cycler shown in more detail in U.S. Patent application S.N. 11/515,359 filed August 31, 2006, entitled "Improved Cassette System for Peritoneal Dialysis Machine. Such a cycler is shown in figures 1A and 1B. In use, a cycler 10 is connected to a number of bags 12 containing dialysis solution. The cycler 10 has a display screen 14, buttons 16, and a cartridge compartment 18. The display screen 14 may be a touch screen, and is used to present a user interface 100 (figures 4A-F). The cartridge compartment 18 accommodates a cartridge 20, shown in figure 2, which is connected to a number of tubes 22 which in turn connect to the bags 12, the patient (not shown), or a drain (not shown).

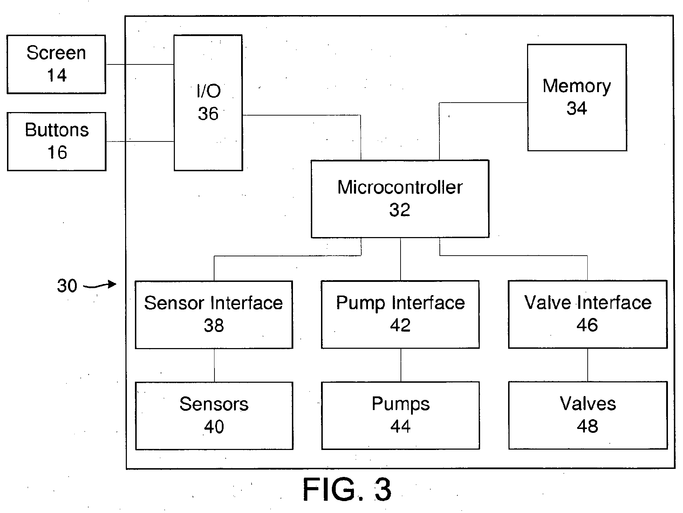

[01] The cycler 10 is controlled by a computer 30, as shown in figure 3. The computer has a microcontroller 32, a memory 34, and an input/output connection 36 to the buttons 16 and display screen 14. A sensor interface 38 connects the microcontroller to sensors 40, a pump interface connects the microcontroller to pumps 44, and a valve interface 46 connects the microcontroller to valves. These three interfaces 38, 42, and 46 allow the computer 30 to operate the cylcer 10 according to software and treatment parameters stored in the memory 34.

In some examples, as shown in figures 4A-F, the user interface 100 includes a series of screens that indicate available options. A first "My Settings" screen, figure 4A, is selected by pressing a button 116 and provides a tab 101a that allows the user to select which therapy type 102 he requires. In the screen shown, CCPD is selected, as indicated by a highlighted box 104, and the other available types are indicated by other boxes 106, 108, 110, 112. Pediatric peritoneal dialysis is beyond the scope of this disclosure. Other buttons 114 and 118 and tabs 101b-d allow the user to configure other sets of settings, some of which are discussed below and others of which are beyond the scope of this disclosure.

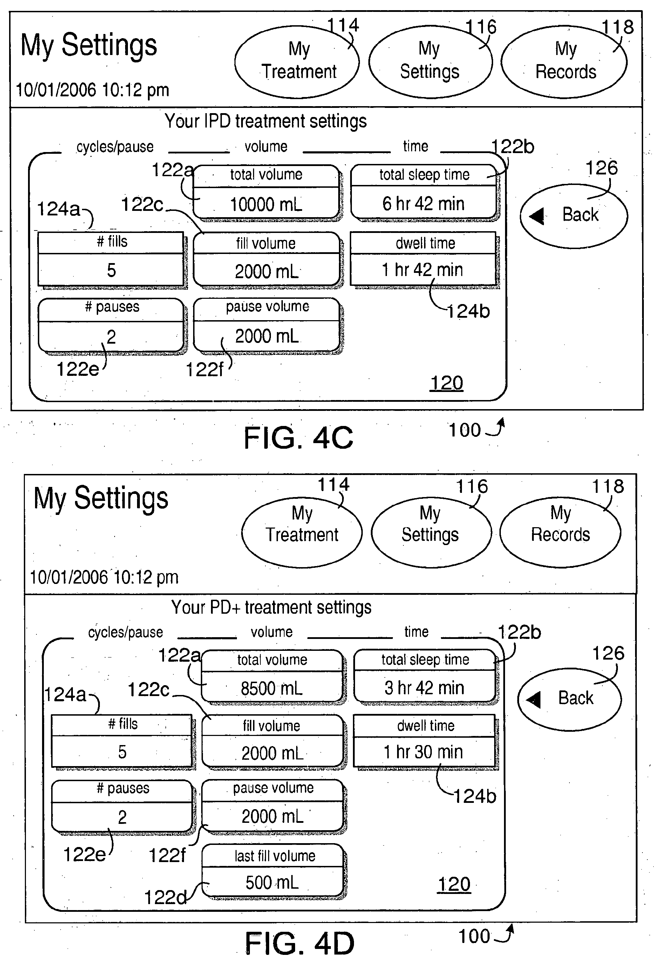

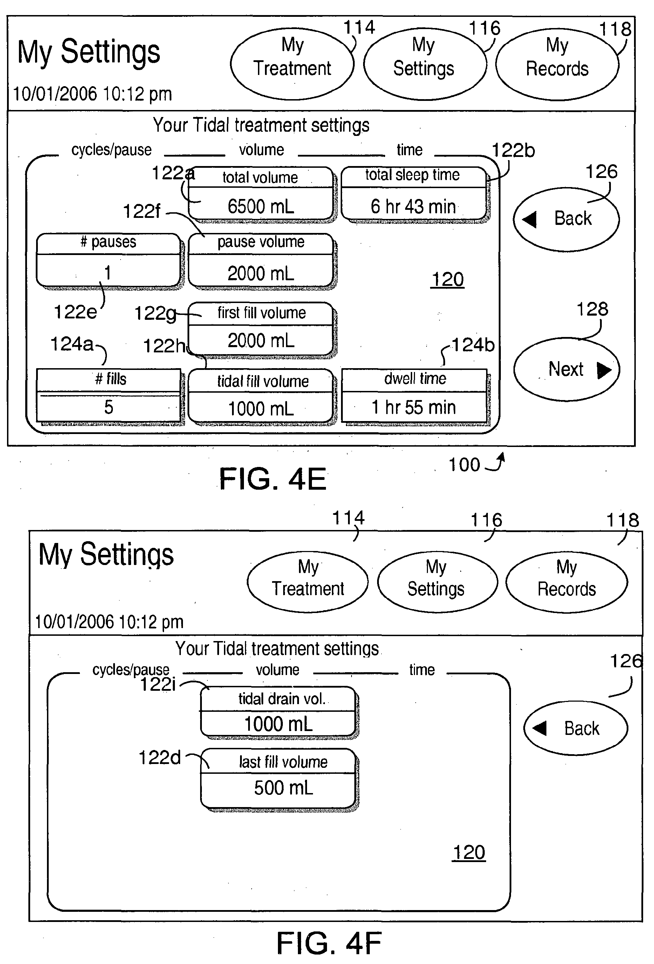

When the user chooses the type of therapy, a screen, figure 4B, showing only the parameters corresponding to the selected therapy (CCPD in figure 4B) is displayed. This screen includes a settings box 120 that makes available the parameters that the user can change for the selected therapy type, in boxes 122a-d, and additional parameters, in boxes 124a-b, that are simply calculated from the values the user enters. In figure 4B, the boxes for either direct or calculated parameter entry and display are differentiated based on their shape, but in practice could be differentiated by color, shading, or other standard user-interface features. Figures 4C, 4D, and 4E-F show corresponding screens for IPD, PD+, and Tidal treatment, respectively. As shown in figure 4C, IPD treatment has two additional user-configurable parameters, entered in boxes 122e-f, but does not have the last fill volume 122d of CCPD treatment. In figure 4D, PD+ has both the pause parameters 122e-f and the last fill volume 122d. In figure 4E, Tidal treatment adds a first fill volume, 122g, and a tidal fill volume 122h, but does not have a per-fill volume 122c. Tidal treatment also has a second screen, figure 4F, for entering the tidal drain volume 122i and the last fill volume 122d. In each of the screens, a back button 126 returns the user to the previous screen - the therapy selection screen of figure 4A for figures 4B-4E, and the first Tidal screen for figure 4F. In figure 4E, a forward button 128 takes to user to the second Tidal screen of figure 4F.

When a user changes the value of any one parameter, the other parameters that are affected by it can be seen instantly. All the parameter values on the screen are updated "on the fly." A comprehensive algorithm can be used to calculate the values as discussed below. This algorithm can be executed in software programmed in the cycler's memory. Validations can be used to make sure that none of the values are out of range and that all the dependencies are satisfied. For example, when the fill volume 122c is changed, the number of fills 124a and the dwell time 124b update automatically according to the calculations. Validation makes sure that, for example, the last fill volume 122d is <=150% of fill volume 122c in treatments that have a fill volume setting. When fill volume 122c is decreased to a value that violates this condition, last fill volume 122d also decreases automatically to ensure that it is always <= 150% of fill volume 122c. In the user interface, when the value of a parameter goes out of range, it can be locked so that the user cannot exceed the limits. For example, the minimum value for total volume 122a may be 50 ml. If a user tries to decrease it below 50 ml, then the value locks at 50 ml and allows the user only to increase it and not decrease it. In another example, a check is made to ensure that there is enough sleep time 122b for the treatment. If the total sleep time 122b is not long enough, then the total therapy volume 122a is locked and cannot be increased unless the total therapy time is increased. This may include locking other settings that would increase total volume 122a, for example, fill volume 122c. Alternatively, if a user decreases the total sleep time 122b, thc system could automatically reduce the fill volume 122c and total volume 122a to accommodate the new sleep time.

This "on the fly" updating gives the user a clear idea of how each parameter value reflects on the others. The user may not be aware of the dependencies but he can still be confident that he is not entering any bad values.

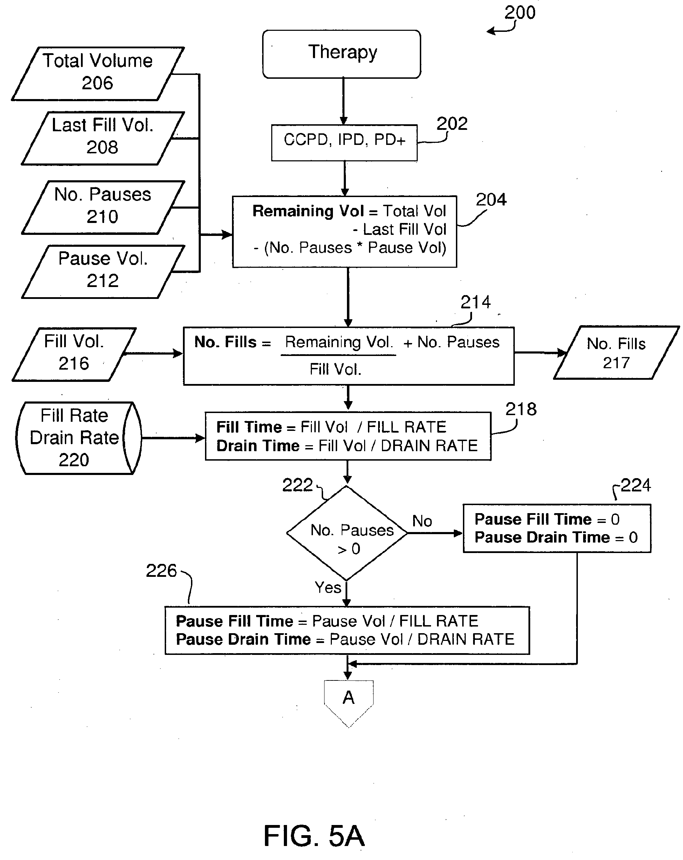

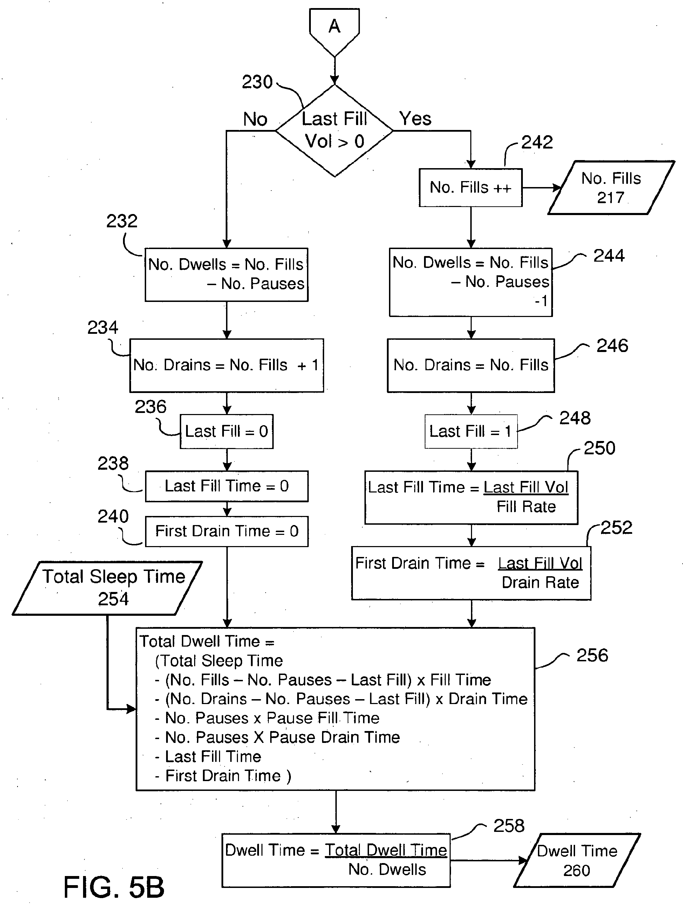

In some examples, parameters for CCPD, IPD, and PD+ are calculated using the process 200 shown in figures 5A and 5B. After the therapy type is selected (202), the remaining volume (the volume that will be used during the regular fills) is calculated (204) based on user inputs of total volume 206, last fill volume 208, the number of pauses 210, and the pause volume. Since not all therapies have pauses, the calculation 204 could be modified accordingly, or the appropriate inputs may be set to zero when not relevant.

Next, in step 214, the remaining volume is divided by the fill volume 216, and the quotient is added to the number of pauses (if any) to determine the number of fills 217. In step 218, the fill volume 216 is divided by the fill rate and drain rate, read from a stored setting 220, to compute the fill time and drain time respectively. If there are no pauses (222), then the pause fill time and drain time are set (224) to zero, otherwise they are computed (226) by dividing the pause volume 212 by the fill rate and drain rate accordingly. Process 200 continues in figure 5B.

If there is no last fill (230) (i.e., the treatment is IPD), the number of dwells is the number of fills minus the number of pauses (232), the number if drains is one more than the number of fills (234), the last fill Boolean is zero (false) (236), and the last fill and first drain times are zero (238, 240). If there is a last fill (230) (i.e., the treatment is CCPD or PD+), the previously calculated number of fills 217 is incremented (242), the number of dwells is set to one less than the number of fills minus the number of pauses (244), the number of drains is set to equal the number or fills (246), the "last fill" Boolean is one (true) (248), and the last fill and first drain times are equal to the last fill volume divided by the fill rate and drain rate, respectively (250, 252).

The time needed for each of the fills and drains is subtracted (256) from the total sleep time 254, to find the total dwell time, which is divided (258) by the number of dwells to find and output the dwell time 260.

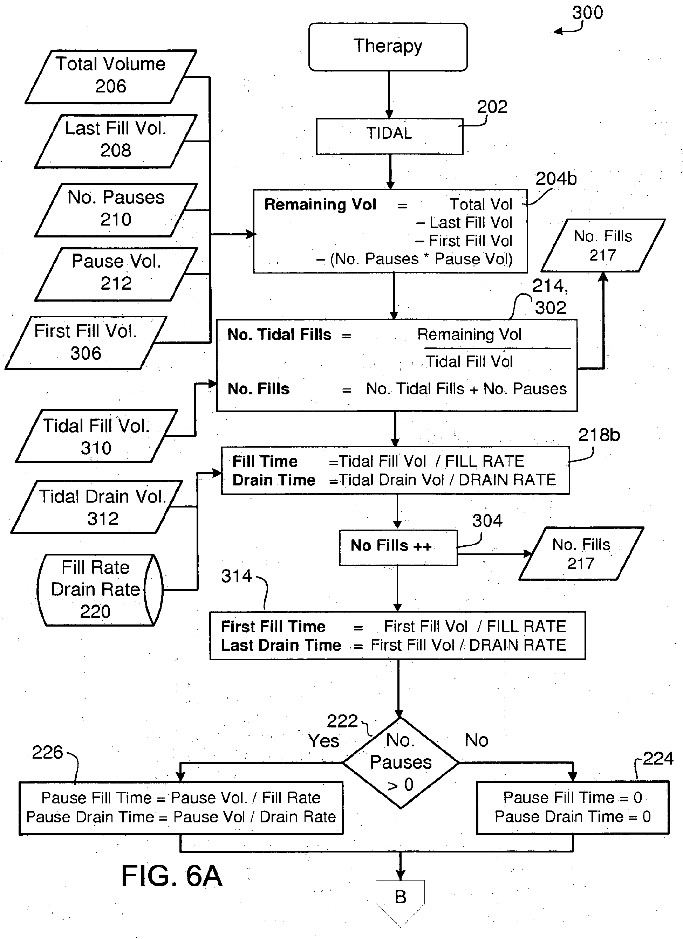

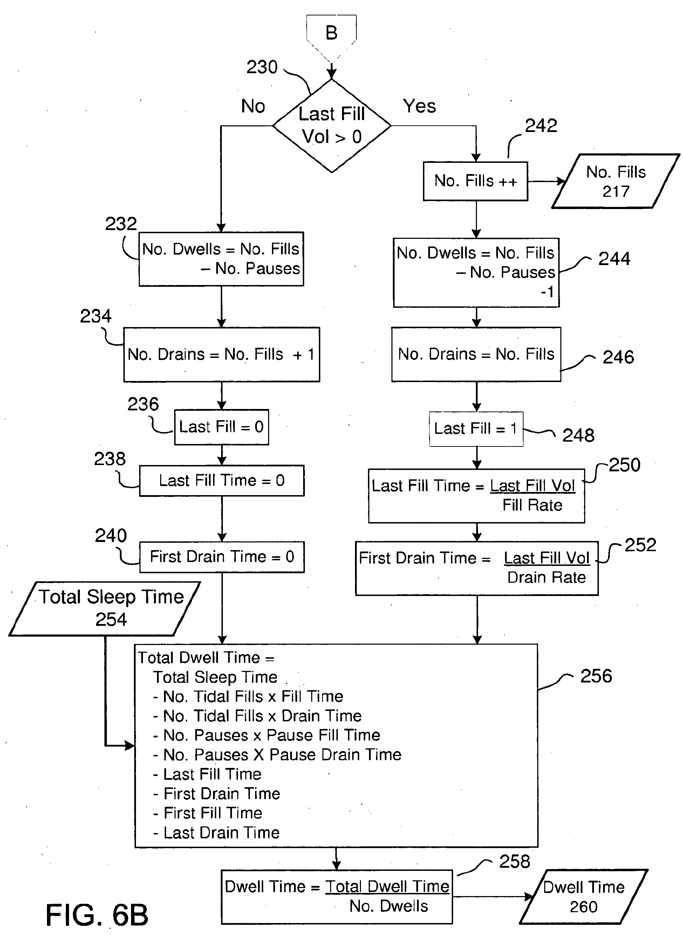

A similar process 300, as shown in figures 6A and 6B, can be used to compute parameters for Tidal therapy. Most of the inputs, steps, and outputs are the same, but a few are modified or reordered, and a few additional inputs and steps are added. The remaining volume calculation 204b subtracts the first fill volume 306 from the total volume in addition to the last fill volume and the total pause volume as before. As a preliminary step to calculating the number of fills (214), the number of tidal fills is calculated (302) based on the remaining volume and the tidal fill volume 310. The tidal fill volume 310 and tidal drain volume 312 are used to calculate the fill time and drain time in step 218b, rather than using a single fill volume for both. An additional step 304 increments the number of fills after the fill time and drain time are calculated, and a first fill time and last drain time are calculated (314) based on the first fill volume 306.

The remaining calculations are the same as in process 200, with the exception of the calculation 256b of total dwell time (figure 6B), which uses the number of tidal fills to find the fill time and drain time rather than subtracting the number of pauses and last fill from the number of fills and drains, and additionally subtracts the first drain time and first fill time.

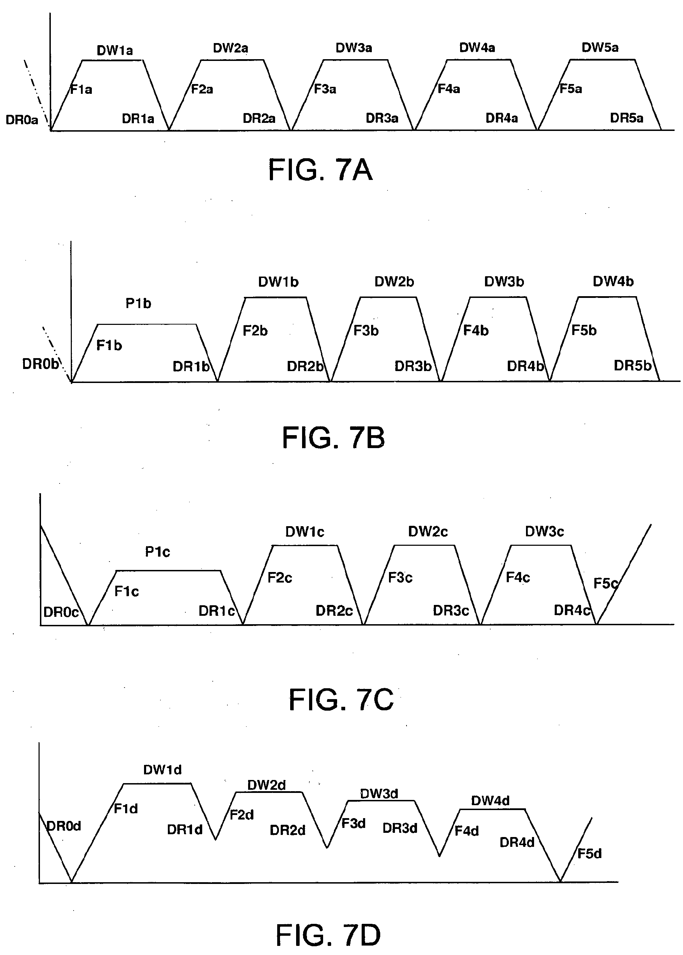

Example parameter calculations for each of the therapy types are described below. Figure 7A shows a graph of the volume of solution in a patient during a CCPD treatment. Upward-sloping segments F1a-F5a are fills, horizontal segments DW1a-DW5a are dwells, and downward-sloping segments DR0a-DR5a are drains, where DR0a is an initial drain to remove any fluid the patient may have from a day time exchange. In a simple example, the user enters the following parameters on box 120 of the screen in figure 4B: total sleep time 90 minutes, total volume 5000 ml, and fill volume 1000 ml, with no last fill or pauses (note these are not the values shown in figure 4B). Using process 200, the remaining volume is calculated in step 204 as 5000 ml - 0 - (0x0) = 5000 ml. This is divided in step 214 by fill volume, 1000 ml, giving number of fills 217 = 5. If the fill rate is 300 ml / min and the drain rate is 200 ml / min, step 218 gives a fill time of 4 minutes (rounding up to whole minutes) and a drain time of 5 minutes. The number of pauses is zero (222) so the pause fill and drain times are zero (224). Because there is no last fill, decision 230 directs the process 200 to the left-hand branch in figure 5B. The number of dwells equals the number of fills, 5 (minus zero), in step 232, and the number of drains is therefore set to 6 in step 234, to account for the DR0a drain. The last fill is false (zero), and last fill and first drain times are zero (if a first drain is necessary, it is not counted in the total dwell time of the treatment). This gives a total dwell time of 90 - 5 x 4 - 6 x 5 = 40 min in step 256 and a dwell time 260 of 8 min when that is divided over the 5 dwells in step 258.

Figure 7B shows a graph of the volume of solution in a patient during an IPD treatment including a pause but no final fill. Fills F1b-F5b, drains DR1b-DR5b, and dwells DW1b-DW4b are as in figure 7A, and segment P1b represents the pause. The difference between the pause and the dwells, as far as the calculations are concerned, is that it doesn't count toward the total dwell time. Modifying the CCPD example above, the pause fill volume is set to 500 ml, and the number of pauses is set to 1. This gives a remaining volume of 4500 in step 204 and a number of fills of 4500/1000 + 1 = 5.5 in step 214. The partial fill is dropped, giving 5 fills for the remaining calculations (see the discussion of parameter validation, below).The fill time is the same as above, and the pause fill and drain times are 500/300 = 2 minutes and 500/200 = 3 min (rounding up) per step 226. Again following the left branch in figure 2B, the number of dwells is 5 - 1 = 4, and the number of drains in 5 + 1 = 6. The total dwell time is 90 - (5 - 1) x 4 - (6 - 1) x 5 - 1 x 2 - 1 x 3 = 44 min, giving a dwell time 260 of 11 min.

Figure 7C shows a graph of the volume of solution in a patient during an PD+ treatment including both a pause and a final fill. The notations follow the same pattern as in the above examples. For this example, the sleep time, total volume, and fill volume remain the same, but pause volume is set to 600 ml and the last fill volume is 1200 ml. Again using process 200, this gives a remaining volume of 3200 ml (step 204) and 3200/1000 + 1 = 4 fills 217 at step 214. Fill time and drain time are again 4 min and 5 min (step 218), and pause fill and drain time are again 2 min and 3 min (this time without rounding). Now following the right-hand branch in figure 5B, the number of fills 217 is incremented to 5 (step 242) to account for the last fill. The number of dwells is 6 - 1 - 1 = 3, per step 244, and the number of drains is 5. The last fill is set true (one - step 248), the last fill time is 1200 / 300 = 4 min (250), and the first drain time is 1200 / 200 = 6 min (252). These values give a total dwell time of 90 - (5 - 1 - 1) x 4 - (5 - 1 - 1) x 5 - 1 x 2 - 1 x 3 - 4 - 6 = 48 min (256) and a dwell time 260 of 48 / 3 = 16 min (258).

An example tidal treatment is shown in figure 7D. The notations are as above, but note that the downward-sloping drain segments DR1d-DR3d and upward-sloping fill segments F2d-F4d don't reach zero between dwells. For this example, total sleep time and total volume are as above. Tidal fill volume is set to 900 ml, with tidal drain volume set to 950 ml. The first fill volume is 1000 ml and the last fill volume is 500 ml. Applying process 300, the remaining volume is 5000 - 500 - 1000 - 0 x 0 = 3500 ml (204b) giving a number of tidal fills of 3500 / 900 = 3 in step 302. Tidal fill time is 900 / 300 = 3 min, while tidal drain time is 950 / 200 = 5 min (218b). The number of fills 217 is incremented by 1 to account for the tidal fill F1d in step 304. The first fill and last drain times are calculated in step 314 as 1000 / 300 = 4 min and 1000 / 200 = 5 min, respectively. Following the right-hand branch in figure 6B, the number of fills 217 is incremented again to account for the last fill (242). The number of dwells is 5 - 0 - 1 = 4 (244) and the number of drains is 5 (246). The last fill Boolean is set to true (one, step 248), and the last fill and first drain times are calculated as 500 / 300 = 2 min (250) and 500 / 200 = 3 min (252), respectively. This all amounts to a total dwell time of 90 - 3 x 3 - 3 x 5 - 0 - 0 - 2 - 3 - 4 - 5 = 52 min (256) and a dwell time 260 of 52 / 4 = 13 min (258).

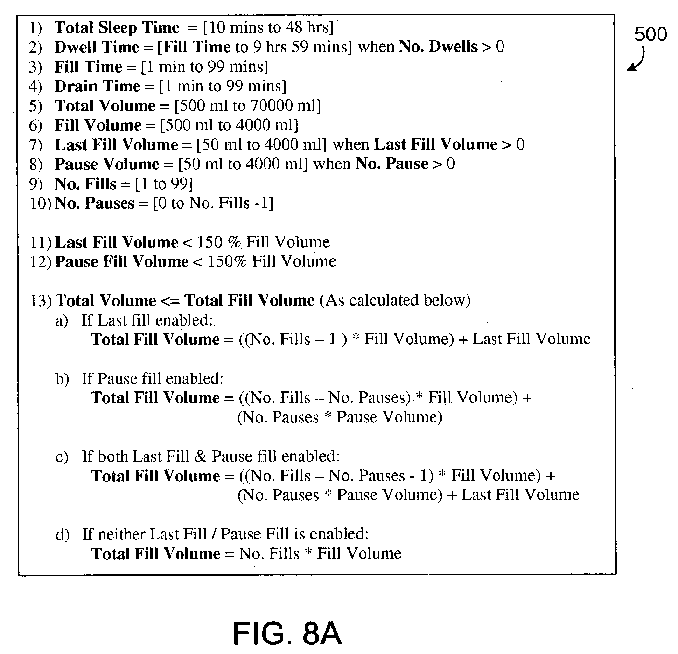

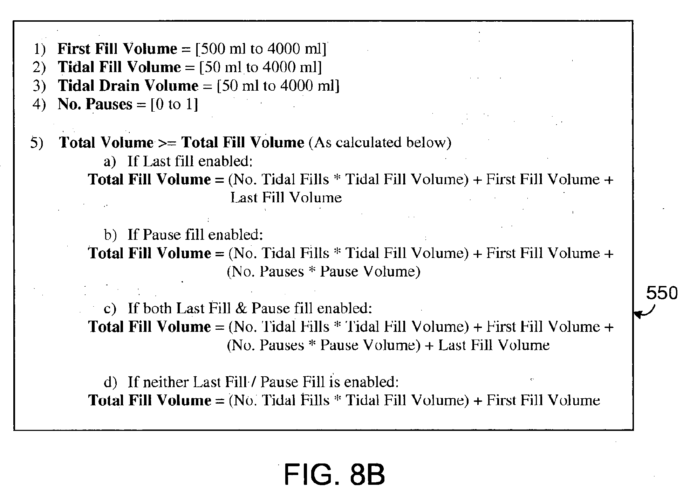

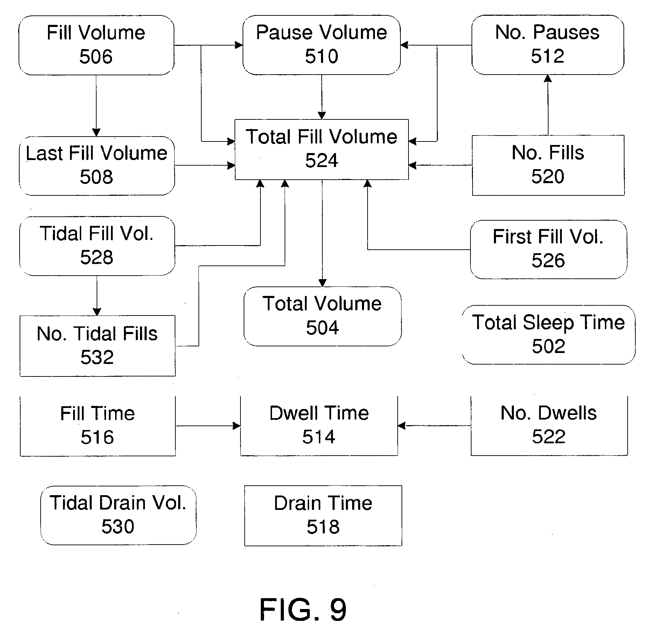

In addition to calculating the number of fills and the dwell time, the system is able to evaluate the validity of each of the input parameters whenever one of them is changed, as mentioned above. A validation table 500 in figure 8A lists several parameter validation relationships. Additional or alternative validations for tidal therapy are shown in figure 8B. The validation relationships between values in tables 500 and 550 are shown graphically in figure 9. In figure 9, user inputs are shown in rounded boxes, and internally computed values are shown in rectangles. Arrows indicate dependency. Elements shown without any arrows into them, e.g., fill time 516 and drain time 518, depend on fixed validation values but not on other input or computed values. Elements that do have arrows in to them may depend on other values, fixed values, or both, as indicated in the validation table 500.

In the example shown, the last fill volume 508 depends on the fill volume 506 according to rule 11 in table 500, that is, last fill volume 508 must be less than 150% of fill volume 506. Similarly, the pause volume 510 must be less than 150% of fill volume 506 according to rule 12. Pause volume is also validated against the number of pauses 512, as pause volume can only have non-zero values when the number of pauses 512 is also non-zero. The number of pauses 512 in turn is validated against the number of fills 520, as its maximum value is one less than the number of fills 520 according to rule 10. The total volume 504 must be less than the total fill volume 524 per rule 13. The total fill volume 524 is not used in the process 200, but is used to validate the total volume input. It is calculated from the fill volume 506, last fill volume 508, pause volume 510, and number of pauses 512 and fills 520 according to rules 13a, b, and c, depending on whether last fill or pauses are enabled (i.e., last fill volume > 0 or number of pauses > 0). The dwell time 514 depends on the fill time 516 and number of dwells 522 according to rule 2. Each of the parameters in figure 9 is also validated against numerical limits per the other rules in table 500.

Applying these tables 500 and 550 to the example tidal therapy calculation above, the total sleep time, total volume, last fill volume, pause volume, and number of fills are all within the fixed numeric ranges of rules 1, 5, 7, 8, and 9. The dwell time is greater than the fill time per rule 2. As for the tidal-specific parameters, the first fill volume, tidal fill volume, tidal drain volume, and number of pauses are all within the ranges of tidal rules 1-4. (The number of pauses also meets the general rule 10 in table 500 as it is zero.) The total volume meets tidal rule 5-a, having a last fill but no pauses, as the total fill volume is 3 x 900 + 1000 + 500 = 3300 ml, and this is less than the total volume of 5000 ml.

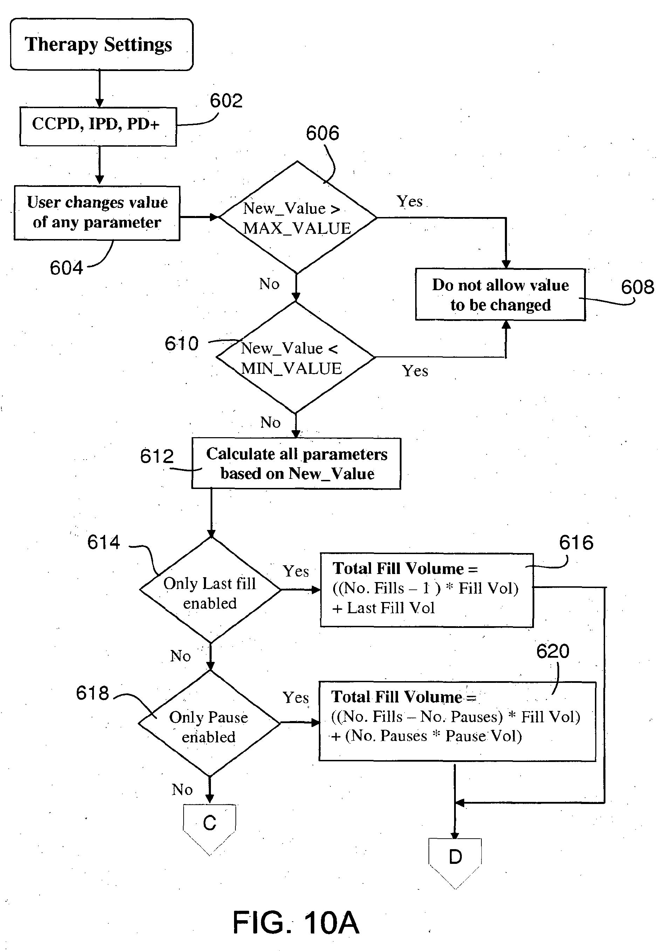

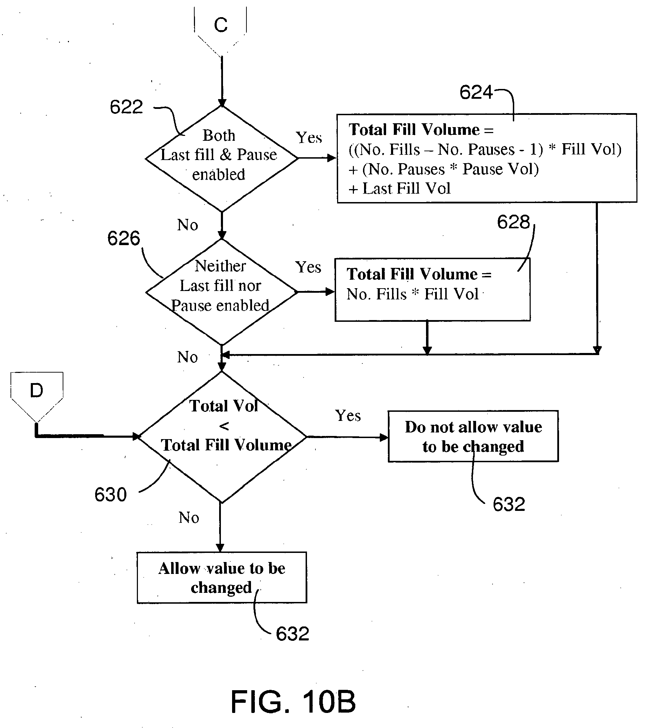

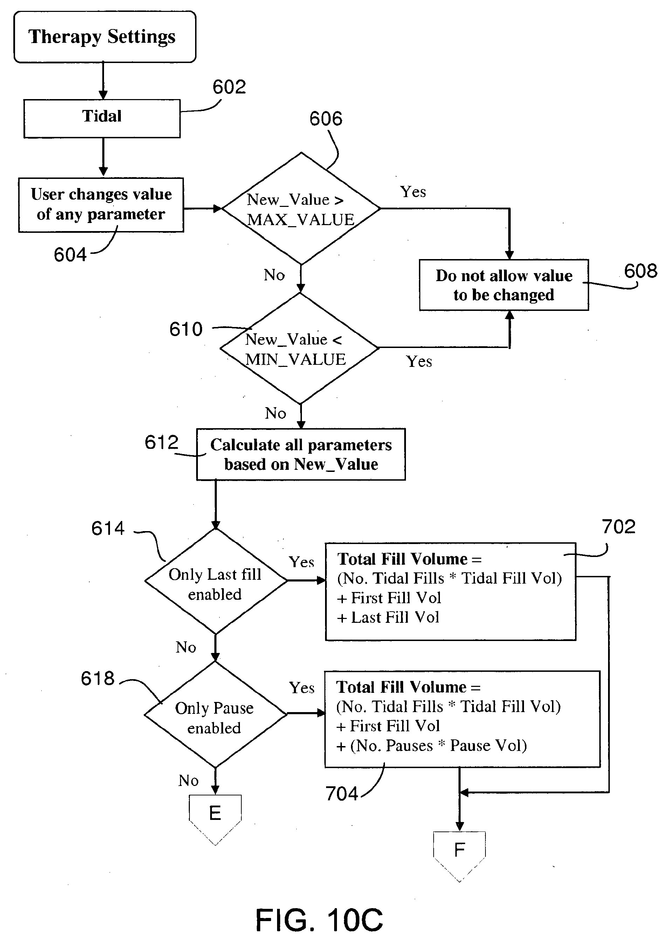

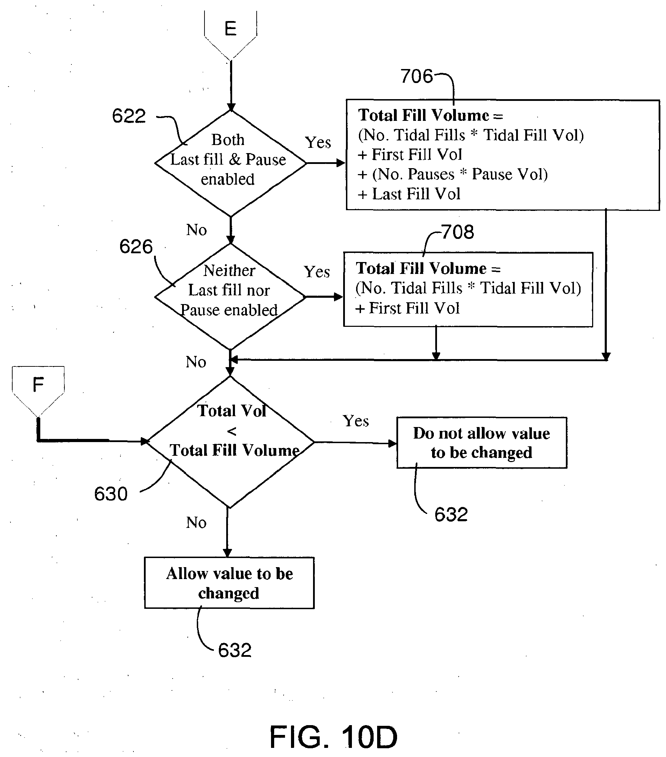

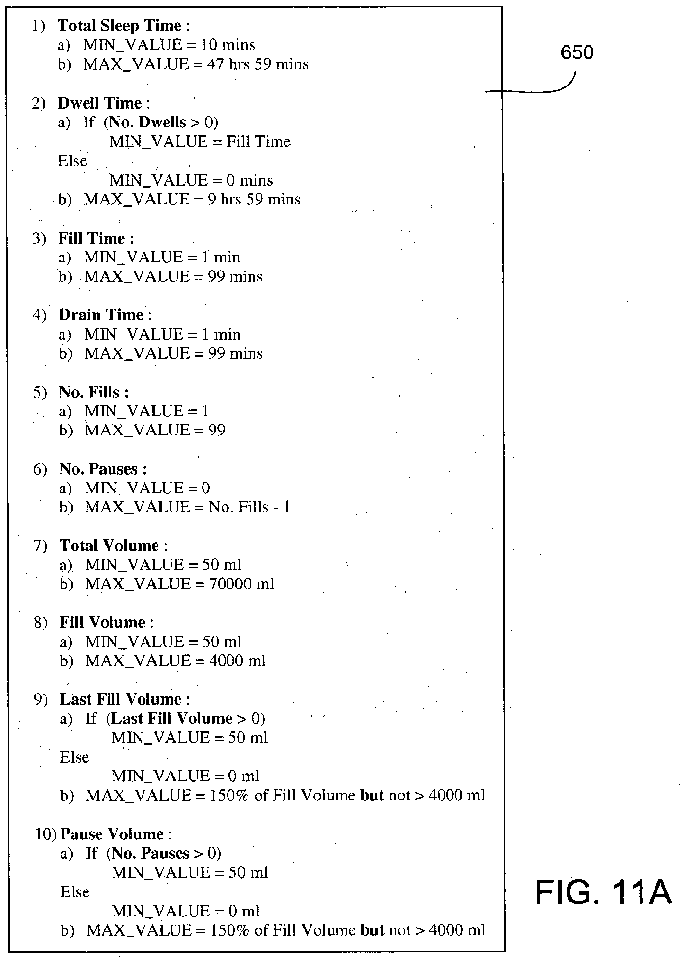

In some examples, a process shown in figures 10A-10D is used to validate parameters as they are entered and assure that they fall within valid ranges. A process 600 in figures 10A and 10B is used for CCPD, IPD, and PD+ treatments, while a process 700 in figure 10C and 10D is used for Tidal treatment. The process 600 is entered when the user selects 602 one of CCPD, IPD, or PD+ treatments and changes 604 the value of any parameter. The process then checks 606 whether the new value is greater than a set maximum value. If it is, the change is not allowed (608). If the new value is less than the maximum, then the process checks 610 whether it is less than a minimum value. Again, if the new value is too low, the change is not allowed (608). A list of minimum and maximum values that may be used by the process 600, based on the rules in table 500 (figure 8A), are shown in table 650 in figure 11A.

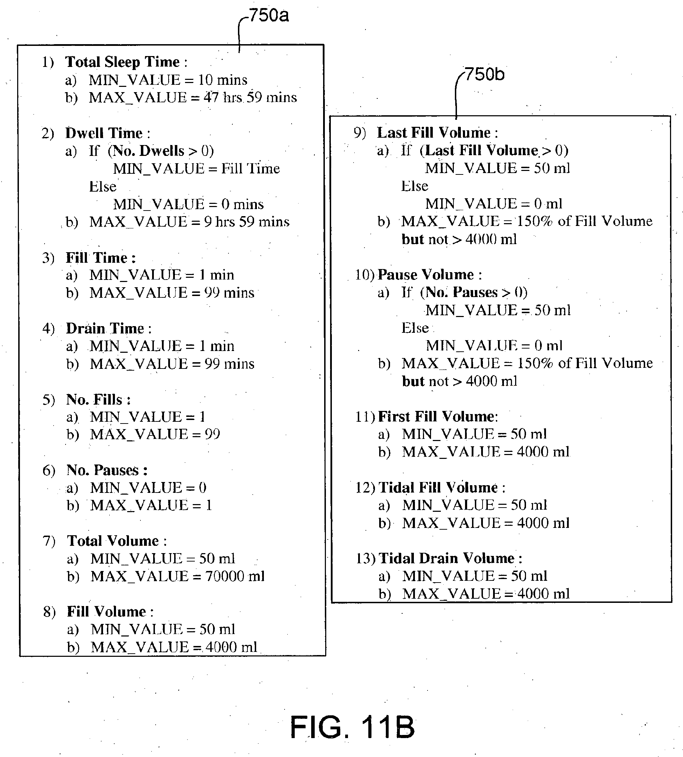

If the new value is between the minimum and maximum values, then the process 600 calculates 612 all the other parameters based on that new value, using the process 200 (figures 5A & 5B) discussed above. If only a last fill is enabled (614), the process 600 calculates 616 the total fill volume based on the number of fills, the fill volume, and the last fill volume. If only a pause is enabled (618), the fill volume is calculated 620 based on the number of fills, number of pauses, fill volume, and pause volume. In either case, the process 600 proceeds (link D) to step 630 discussed below. If both conditions 614 and 618 are not true, the process 600 proceeds (link C) to step 622. If both last fill and pause are enabled, the total fill volume is calculated 624 based on the number of fills and pauses, the fill volume and pause volume, and the last fill volume. If neither last fill nor pause are enabled (626), then the total fill volume is calculated 628 based only on the number of fills and the fill volume. After the total fill volume is calculated in steps 616, 620, 624, or 628, it is compared 630 to the total volume entered by the user. If the total fill volume would be larger than the total volume, the change is not allowed (632). If the total fill volume is less than the total volume, the value is changed (634). The process 700 is essentially the same, with steps 616, 620, 624, and 628 replaced by steps 702, 704, 706, and 708 to calculate the total fill volume based on the number of tidal fills, tidal fill volume, and first fill volume. Minimum and maximum values for use by process 700 are shown in table 750a and 750b in figure 11B.

Other implementations are within the scope of the following claims.

Although the present invention has been described above, and is defined in the appended claims. The following embodiments are given as examples :
1. A method of setting parameter values in a peritoneal dialysis device comprising:
   receiving from a user a selection of a therapy type,
   receiving from the user a value for an input parameter, and
   automatically setting a value for a number of fills parameter or a dwell time parameter based on the value received from the user.
2. The method of embodiment 1 in which setting a value for the number of fills or dwell time parameter comprises:
   calculating a value for the number of fills or dwell time, and
   assigning the calculated value to the parameter.
3. The method of embodiment 1 in which setting a value for the number of fills or dwell time parameter comprises:
   calculating an updated value for the number of fills or dwell time,
   determining that the updated value does not meet a criterion, and
   assigning a value to a third parameter.
4. The method of embodiment 3 also comprising:
   calculating an updated value for the number of fills or dwell time based on the value assigned to the third parameter, and
   assigning the updated value to the number of fills or dwell time parameter.
5. The method of embodiment 3 in which the criterion is that the updated value is within a pre-set range.
6. The method of embodiment 3 in which the criterion is that the updated value has a specified relationship to a fourth parameter.
7. The method of embodiment 1 also comprising:
   communicating the value set for the number of fills or dwell time parameter to the user, and
   communicating to the user a relationship between the value received from the user and the value set for the number of fills or dwell time parameter.
8. A method of setting parameter values in a peritoneal dialysis device comprising:
   receiving from a user a selection of a therapy type,
   receiving from a user values for a plurality of parameters,
   calculating a value for a plurality of additional parameters based on the values received from the user, the plurality of additional parameters comprising a number of fills parameter or a dwell time parameter,
   determining that values for one or more of the plurality of parameters or one or more of the additional parameters do not meet one or more criteria, and
   updating one or more of the values received from the user so that all of the values for the plurality of parameters and the additional parameters meet all the criteria.
9. A method of setting parameter values in a peritoneal dialysis device comprising:
   displaying on a graphical interface a plurality of parameters and any values currently assigned to the parameters,
   in response to a user selecting one of the displayed parameters, allowing the user to input a value to be assigned to the parameter,
   calculating updated values for one or more parameters based on the value input by the user and assigning the updated values to the corresponding parameters,
   determining whether values assigned to one or more of the parameters meet one or more criteria, and
   if the values do not meet the criteria, calculating one or more further updated values for other parameter values that will cause a value that does not meet a criterion to meet the criterion and assigning the further updated values to the corresponding parameters.
10. A method of setting parameter values in a peritoneal dialysis device comprising:
   receiving an instruction from a user to change a value of a parameter to a new value,
   determining whether the new value is greater than a maximum value for the parameter,
   if the new value is greater than the maximum value, rejecting the new value,
   if the new value is less than the maximum value, determining whether the new value is less than a minimum value for the parameter, and
   if the new value is less than the minimum value, rejecting the new value.
11. The method of embodiment 10 further comprising:
   if the new value is greater than the minimum value, calculating an updated value for a second parameter based on the new value,
   calculating a total fill volume,
   determining whether the total fill volume is greater than a total treatment volume,
   if the total fill volume is greater than the total treatment volume, rejecting the new value, and
   if the total fill volume is less than the total treatment volume, changing the value of the parameter to the new value.
12. A computer readable medium containing instructions to cause a peritoneal dialysis device to:
   display on a graphical interface a plurality of parameters and any values currently assigned to the parameters,
   in response to a user selecting one of the displayed parameters, allow the user to input a value to be assigned to one of the parameters,
   calculate updated values for one or more additional parameters based on the value input by the user and assign the updated values to the corresponding parameters,
   determine whether values assigned to one or more of the additional parameters meet one or more criteria, and
   if the values do not meet the criteria, calculate one or more further updated values for other parameter values that will cause a value that does not meet a criterion to meet the criterion and assign the further updated values to the corresponding parameters.
13. The medium of embodiment 12 in which the instructions also cause the device to:
   display the updated values on the graphical interface.
14. The medium of embodiment 12 in which the instructions also cause the device to:
   determine that values assigned to one or more of the additional parameters do not meet one or more criteria, and
   inform the user that the input value is rejected.
15. The medium of embodiment 12 which the instructions also cause the device to perform peritoneal dialysis fills and drains based on the values assigned to the parameters.
16. The medium of embodiment 12 in which the instructions also cause the device to:
   determine that the value input by the user does not meet one or more criteria, and
   inform the user that the input value is rejected.
17. A computer readable medium containing instructions to cause a peritoneal dialysis device to:
   receive from a user a selection of a therapy type,
   receive from the user a value for an input parameter, and
   automatically set a value for a number of fills parameter or a dwell time parameter based on the value received from the user.
18. The medium of embodiment 17 in which the instructions also cause the device to:
   calculate a value for the number of fills or dwell time, and
   assign the calculated value to the parameter.
19. A peritoneal dialysis cycler comprising a control computer configured to cause the cycler to:
   display on a graphical interface a plurality of parameters and any values currently assigned to the parameters,
   in response to a user selecting one of the displayed parameters, allow the user to input a value to be assigned to one of the parameters,
   calculate updated values for one or more additional parameters based on the value input by the user and assign the updated values to the corresponding parameters,
   determine whether values assigned to one or more of the additional parameters meet one or more criteria, and
   if the values do not meet the criteria, calculate one or more further updated values for other parameter values that will cause a value that does not meet a criterion to meet the criterion and assign the further updated values to the corresponding parameters.
20. The cycler of embodiment 19 in which the control computer is also configured to cause the device to:
   display the updated values on the graphical interface.
21. The cycler of embodiment 19 in which the control computer is also configured to cause the device to:
   determine that values assigned to one or more of the additional parameters do not meet one or more criteria, and
   inform the user that the input value is rejected.
22. The cycler of embodiment 19 which the control computer is also configured to cause the device to perform peritoneal dialysis fills and drains based on the values assigned to the parameters.
23. The cycler of embodiment 19 in which the control computer is also configured to cause the device to:
   determine that the value input by the user does not meet one or more criteria, and
   inform the user that the input value is rejected.
24. The cycler of embodiment 19 also comprising the graphical interface.
25. The cycler of embodiment 24 in which the graphical interface comprises a touch-sensitive display screen.
26. A peritoneal dialysis cycler comprising a control computer configured to cause the cycler to
   receive from a user a selection of a therapy type,
   receive from the user a value for an input parameter, and
   automatically set a value for a number of fills parameter or a dwell time parameter based on the value received from the user.
27. The cycler of embodiment 26 in which the control computer is also configured to cause the device to:
   calculate a value for the number of fills or dwell time, and
   assign the calculated value to the parameter.

## Claims

1. A method of setting parameter values in a peritoneal dialysis device comprising:
receiving an instruction from a user to change a value of a parameter to a new value,
determining whether the new value is greater than a maximum value for the parameter,
if the new value is greater than the maximum value, rejecting the new value,
if the new value is less than the maximum value, determining whether the new value is less than a minimum value for the parameter, and
if the new value is less than the minimum value, rejecting the new value; and
if the new value is greater than the minimum value, calculating an updated number of fills, an updated dwell time and an updated value for a total fill volume parameter based at least in part on the new value, the calculating comprising determining whether neither, only one or both of a last fill parameter and a pause parameter are enabled; and
determining whether the total fill volume is greater than a total treatment volume entered by a user,
if the total fill volume is greater than the total treatment volume, rejecting the new value, and
if the total fill volume is less than the total treatment volume, changing the value of the parameter to the new value.

2. The method of claim 1, wherein calculating an updated value for a total fill volume parameter is based at least in part on a number of fills, a fill volume, and a last fill volume if only the last fill parameter is enabled.

3. The method of claim 1, wherein calculating an updated value for a total fill volume parameter is based at least in part on a number of fills, a fill volume, a number of pauses and a pause volume if only the pause parameter is enabled.

4. The method of claim 1, wherein calculating an updated value for a total fill volume parameter is based at least in part on a number of fills, a fill volume, a number of pauses, a pause volume and a last fill volume if both the pause parameter and the last fill parameter are enabled.

5. The method of claim 1, wherein calculating an updated value for a total fill volume parameter is based at least in part on a number of fills and if both the pause parameter and the last fill parameter are not enabled.

6. The method of claim 1, wherein the parameter comprises one or more of a fill volume, a number of fills, a last fill volume, a number of pauses, a pause volume, a tidal fill volume and a tidal drain volume.

7. The method of claim 1, further including displaying the updated number of fills, an updated dwell time and an updated value for a total fill volume parameter.

## Patentansprüche

1. Verfahren zum Einstellen von Parameterwerten an einer Peritonealdialysevorrichtung, umfassend:
Empfangen einer Anweisung von einem Benutzer, einen Wert eines Parameters zu einem neuen Wert zu verändern,
Bestimmen, ob der neue Wert größer als ein Maximalwert für den Parameter ist,
wenn der neue Wert größer als der Maximalwert ist, Ablehnen des neuen Werts,
wenn der neue Wert kleiner als der Maximalwert ist, Bestimmen, ob der neue Wert kleiner als ein Minimalwert für den Parameter ist, und
wenn der neue Wert kleiner als der Minimalwert ist, Ablehnen des neuen Werts; und
wenn der neue Wert größer als der Minimalwert ist, Berechnen einer aktualisierten Anzahl von Füllungen, einer aktualisierten Verweildauer und eines aktualisierten Werts für einen Gesamtfüllvolumen-Parameter wenigstens zum Teil auf der Grundlage des neuen Werts, wobei das Berechnen das Bestimmen davon umfasst, ob keine, nur eine oder beide von einem letzte-Füllung-Parameter und einem Pausenparameter aktiviert sind; und
Bestimmen, ob das Gesamt-Füllvolumen größer als ein von einem Benutzer eingegebenes Gesamt-Behandlungsvolumen ist,
wenn das Gesamt-Füllvolumen größer als das Gesamt-Behandlungsvolumen ist, Ablehnen des neuen Werts, und
wenn das Gesamt-Füllvolumen kleiner als das Gesamt-Behandlungsvolumen ist, Verändern des Werts des Parameters zu dem neuen Wert.

2. Verfahren gemäß Anspruch 1, wobei die Berechnung eines aktualisierten Werts für einen Gesamtfüllvolumen-Parameter wenigstens zum Teil auf der Grundlage einer Anzahl von Füllungen, eines Füllvolumens und eines letzten Füllvolumens steht, wenn nur der letzte-Füllung-Parameter aktiviert ist.

3. Verfahren gemäß Anspruch 1, wobei die Berechnung eines aktualisierten Werts für einen Gesamtfüllvolumen-Parameter wenigstens zum Teil auf der Grundlage einer Anzahl von Füllungen, eines Füllvolumens, einer Anzahl von Pausen und eines Pausenvolumens steht, wenn nur der Pausenparameter aktiviert ist.

4. Verfahren gemäß Anspruch 1, wobei die Berechnung eines aktualisierten Werts für einen Gesamtfüllvolumen-Parameter wenigstens zum Teil auf der Grundlage einer Anzahl von Füllungen, eines Füllvolumens, einer Anzahl von Pausen, eines Pausenvolumens und eines letzten Füllvolumens steht, wenn sowohl der Pausenparameter als auch der der letzte-Füllung-Parameter aktiviert sind.

5. Verfahren gemäß Anspruch 1, wobei die Berechnung eines aktualisierten Werts für einen Gesamtfüllvolumen-Parameter wenigstens zum Teil auf der Grundlage einer Anzahl von Füllungen steht, wenn sowohl der Pausenparameter als auch der der letzte-Füllung-Parameter nicht aktiviert sind.

6. Verfahren gemäß Anspruch 1, wobei der Parameter eines oder mehrere von einem Füllvolumen, einer Anzahl von Füllungen, einem letzten Füllvolumen, einer Anzahl von Pausen, einem Pausenvolumen, einem Tidal-Füllvolumen und einem Tidal-Entleerungsvolumen umfasst.

7. Verfahren gemäß Anspruch 1, ferner umfassend das Anzeigen der aktualisierten Anzahl von Füllungen, einer aktualisierten Verweildauer und eines aktualisierten Werts für einen Gesamtfüllvolumen-Parameter.

## Revendications

1. Procédé de réglage de valeurs de paramètres dans un dispositif de dialyse péritonéale comprenant les étapes suivantes :
recevoir d'un utilisateur une instruction pour changer une valeur d'un paramètre en une nouvelle valeur,
déterminer si la nouvelle valeur est supérieure à une valeur minimale pour le paramètre,
si la nouvelle valeur est supérieure à la valeur maximale, rejeter la nouvelle valeur,
si la nouvelle valeur est inférieure à la valeur maximale, déterminer si la nouvelle valeur est inférieure à une valeur minimale pour le paramètre, et
si la nouvelle valeur est inférieure à la valeur minimale, rejeter la nouvelle valeur, et
si la nouvelle valeur est supérieure à la valeur minimale, calculer un nombre de remplissages mis à jour, un temps de séjour mis à jour et une valeur mise à jour pour un paramètre de volume de remplissage total sur la base, au moins en partie, de la nouvelle valeur,
le calcul comprenant de déterminer si ni l'un, ni l'autre, uniquement un seul, ou les deux, parmi un paramètre de dernier remplissage et un paramètre de pause sont activés ; et
déterminer si le volume de remplissage total est supérieur à un volume de traitement total entré par un utilisateur,
si le volume de remplissage total est supérieur au volume de traitement total, rejeter la nouvelle valeur, et
si le volume de remplissage total est inférieur au volume de traitement total, changer la valeur du paramètre en la nouvelle valeur.

2. Procédé selon la revendication 1, dans lequel le calcul d'une valeur mise à jour pour un paramètre de volume de remplissage total est basé, au moins en partie, sur un nombre de remplissages, un volume de remplissage, et un volume de dernier remplissage si uniquement le paramètre de dernier remplissage est activé.

3. Procédé selon la revendication 1, dans lequel le calcul d'une valeur mise à jour pour un paramètre de volume de remplissage total est basé, au moins en partie, sur un nombre de remplissages, un volume de remplissage, un nombre de pauses et un volume de pause si uniquement le paramètre de pause est activé.

4. Procédé selon la revendication 1, dans lequel le calcul d'une valeur mise à jour pour un paramètre de volume de remplissage total est basé, au moins en partie, sur un nombre de remplissages, un volume de remplissage, un nombre de pauses, un volume de pause et un dernier volume de remplissage si à la fois le paramètre de pause et le paramètre de dernier remplissage sont activés.

5. Procédé selon la revendication 1, dans lequel le calcul d'une valeur mise à jour pour un paramètre de volume de remplissage total est basé, au moins en partie, sur un nombre de remplissages et sur le fait qu'à la fois le paramètre de pause et le paramètre de dernier remplissage ne soient pas activés.

6. Procédé selon la revendication 1, dans lequel le paramètre comprend un ou plusieurs paramètres parmi un volume de remplissage, un nombre de remplissage, un volume de dernier remplissage, un nombre de pauses, un volume de pause, un volume de remplissage de flux et un volume de drain de flux.

7. Procédé selon la revendication 1, comprenant en outre d'afficher le nombre mis à jour de remplissages, un temps de séjour mis à jour et une valeur mise à jour pour un paramètre de volume de remplissage total.
